(19) Europäisches Patentamt
European Patent Office
Office européen des brevets

(11) **EP 0 770 624 B1**

(12) **EUROPEAN PATENT SPECIFICATION**

(45) Date of publication and mention
of the grant of the patent:
**02.05.2003 Bulletin 2003/18**

(51) Int Cl.[7]: **C07K 14/705**, A61K 39/00,
C12N 15/12, C07K 7/06

(21) Application number: **96307163.4**

(22) Date of filing: **30.09.1996**

(54) **Peptide capable of inducing immune response to human gastric cancer and agent containing it for preventing or treating human gastric cancer**

Peptid das eine Immunantwort gegen menschlichen Magenkrebs induziert und Arzneimittel die es enthält zur Vorbeugung oder Behandlung von Magenkrebs

Peptide pouvant induire une réponse immune contre le cancer de l'estomac et agent contenant ce peptide pour la prévention ou le traitement du cancer de l'estomac

(84) Designated Contracting States:
**BE CH DE ES FR GB IT LI NL SE**

(30) Priority: **29.09.1995 JP 25349195**
**19.08.1996 JP 21714096**

(43) Date of publication of application:
**02.05.1997 Bulletin 1997/18**

(73) Proprietors:
• **Ajinomoto Co., Inc.**
**Tokyo 104 (JP)**
• **Kikuchi, Kokichi**
**Sapporo-shi, Hokkaido, Tokyo (JP)**

(72) Inventors:
• **Kikuchi, Kokichi**
**Chuo-ku, Sapporo-shi, Hokkaido (JP)**
• **Sato, Noriyuki**
**Toyohira-ku, Sapporo-shi, Hokkaido (JP)**
• **Sahara, Hiromitsu**
**Rishirifuji-machi, Rishiri-gun, Hokkaido (JP)**
• **Yasojima, Takahiro**
**Setana-gun, Hokkaido (JP)**

• **Wada, Yoshimasa**
**Chuo-ku, Sapporo-shi, Hokkaido (JP)**
• **Suzuki, Manabu, c/o Central Research Labs**
**Kawasaki-shi, Kanagawa-ken (JP)**
• **Hamuro, Junji, c/o Central Research Labs**
**Kawasaki-ku, Kawasaki-shi, Kanagawa-ken (JP)**

(74) Representative: **Nicholls, Kathryn Margaret et al**
**MEWBURN ELLIS**
**York House**
**23 Kingsway**
**London WC2B 6HP (GB)**

(56) References cited:
• SHU QIN LIU ET AL: "INDUCTION OF HUMAN AUTOLOGOUS CYTOTOXIC T LYMPHOCYTES ON FORMALIN-FIXED AND PARAFFIN-EMBEDDED TUMOUR SECTIONS" NATURE MEDICINE, vol. 1, no. 3, March 1995, pages 267-271, XP002050775
• YASOSHIMA T.: "Analysis of Tumor Rejection of a Gastric Cancer Recognized by Human Autologous Tumor-Specific CTL" BIOTHERAPY, vol. 9, no. 5, May 1995, pages 667-8, XP002081947

**Description**

Field of the invention

The present invention relates to a peptide capable of inducing cytotoxic T lymphocytes (CTL) (1) to human gastric cells in vivo or in vitro, and to a DNA encoding this peptide. More specifically, the present invention relates to a peptide capable of presenting CTL to human gastric cells by being bound to HLA-A31 antigen (2), and to a DNA encoding the peptide. Further, the present invention relates to an agent for preventing or treating a human gastric cancer, the agent containing a peptide capable of inducing CTL to a human gastric cancer cell in vivo or in vitro, and to a vaccine for preventing or treating a human gastric cancer, the vaccine containing a recombinant virus or a recombinant bacterium having a DNA encoding this peptide.

Background to the invention

For pharmacotherapy of malignant tumors, a chemotherapeutic agent which directly impairs tumour cells, or an immunotherapeutic agent with which treatment is conducted by non-specifically activating an immunity of a host and enhancing a bioprotective function of the host has been used. However, there is currently no agent with which malignant tumours, above all, digestive tumours can completely be cured.

In recent years, researches using tumours of animals, mainly mice, have revealed that tumours can completely be cured by enhancing an antigen-specific immune response to tumour-related antigens and tumour-specific antigens present in various tumour cells. Clinically, the treatment has been conducted by enhancing the antigen-specific immune response to these tumour-specific antigens.

With respect to agents or approaches by which tumours are treated upon enhancing the tumour antigen-specific immune response, it has been reported that a monoclonal antibody against an antigen which is expressed in tumour cells and mainly recognized by B-cells is used, that a tumour-specific immune response is induced by administering to the patients their own tumour cells or solubilized fractions thereof which have been inactivated with radiation or medicines as a vaccine, and that in order to increase an immunogenicity of tumour cells, viruses or various cytokine genes are introduced into tumour cells and the thus-treated tumour cells are administered to the patients themselves, whereby a tumour-specific immune response is induced.

However, it is being made clear that T-cells including CTL mainly act on tumour rejection in vivo. It has been currently clarified that treatment using an antibody against a B-cell recognition antigen is limited.

Further, it has been clarified that the immune response with the T-cells acts either intensively or suppressively in a state where two functional sub-populations (Th1 and Th2 subsets) present in T-cell populations are activated, and that the immune reaction produced by tumour cells themselves containing plural antigens sometimes rather suppresses the immune response to the tumour cells.

Still further, with respect to inoculation with inactivated tumour cells, the possibility of re-growth of tumours through re-activation in vivo cannot be completely discounted. Thus, the problem of safety remains.

In order to try and avoid the above-mentioned problems, a method has been proposed in which an antigen protein to activate CTL which is considered to play a central role in tumour rejection is identified, and CTL is activated using this antigen protein.

It has been clarified through research in recent years that CTL is induced by a peptide of about 8 to 12 amino acids, derived by protease fragmentation of an antigenic protein, and which acts as an antigen-presenting molecule to CTL by being bound to an HLA antigen.

Accordingly, in relation to a cancer antigen peptide, it is considered that if a peptide which is bound to an HLA molecule so as to induce CTL to tumour cells can be discovered, this peptide can be used as an agent for preventing or treating cancers.

However, to date, only a peptide derived from a protein of the MAGE family, Mart-1, Thyrosinase, gpl00, which is a cancer antigen present in melanoma tumours, has been identified. As regards cancers of the digestive system, including gastric cancer, the presence of a cancer antigen peptide capable of inducing CTL, and the structure of such a cancer antigen peptide, are currently unknown.

The present invention seeks to provide: (i) a peptide capable of inducing an immune response to a human gastric cancer, (ii) DNA encoding this peptide, (iii) an agent containing the above peptide for preventing or treating a human gastric cancer, (iv) a vaccine for preventing or treating a human gastric cancer, the vaccine containing a recombinant virus or a recombinant bacterium having the DNA encoding the above-mentioned peptide.

Liu et al. (1995) Nature Medicine 1(3):267-271 reports that human autologous tumour-specific cytotoxic T lymphocytes (CTL) were generated from peripheral blood on small formalin-fixed parafin-embedded sections of a gastric cancer.

Yasoshima et al. (1995) Biotherapy 9(5):667-668 reports the elucidation of the cytotoxic mechanism in a human gastric cancer. A MHC class I-restricted CTL clone, TcHST-2, and the autologous gastric signet ring cell carcinoma line, HST-2 were established.

## Summary of the invention

The present inventors have focused on the fact that CTL which recognizes the tumour antigen plays an important role as a bioprotective mechanism to tumour cells. That is, they have conducted studies having in mind that the efficient induction of CTL with a peptide which can be used as a part of a vaccine, or with a transformant containing DNA encoding the peptide, may be effective for preventing or treating cancers.

It is known that the HLA antigen is bound to the cancer antigen peptide and acts as an antigen-presenting molecule to CTL whereby CTL is induced. Accordingly, it is considered that if a peptide which can activate CTL reactive with gastric cancer cells can be found, it may be possible to use it as an agent for preventing or treating the gastric cancer.

The present inventors have succeeded in establishing a CTL cell strain (Tc-HST-2) which is reacts specifically with gastric cancer cells restricted by HLA-A31, this strain being derived from a patient suffering from gastric cancer, and they have succeeded in establishing a gastric cancer cell strain (HST-2) on which this CTL acts, this strain being derived from the same patient (3).

However, it has been unclear whether this CTL recognizes a tumour antigen, and if so what antigen this CTL recognizes. On top of that, the presence of a cancer antigen peptide which exists specifically in digestive cancers including a gastric cancer, and which is capable of inducing CTL, has not been identified at all; much less has it been characterised.

Now, however, the present inventors have purified an HLA-bound peptide present on the cell surface of gastric cancer cell HST-2, and have identified a peptide that activates CTL cell strain Tc-HST-2. Further, they have chemically synthesized this identified peptide in the usual manner. Thus, it has been established for the first time that this peptide actually activates the CTL cell strain Tc-HST-2. It has also been found that this peptide is expressed in gastric cancer cells other than HST-2.

From these results, it is evident that the above-mentioned peptide can be utilized as an agent for preventing or treating gastric cancer. That is, the present invention relates to: (i) a peptide as defined in the appended claims which is a fragment of a gastric cancer antigen protein present in a human gastric cancer cell, the fragment being bound to an HLA molecule and capable of inducing a cytotoxic T-cell that targets the gastric cancer cell; (ii) an agent for preventing or treating a human gastric cancer, the agent containing the above-mentioned peptide; (iii) DNA encoding the above-mentioned peptide; and (iv) a vaccine for preventing or treating a human gastric cancer, the vaccine containing a recombinant virus or a recombinant bacterium having this DNA.

## Brief Description of the Drawings

Fig. 1 is an elution pattern of an HLA-bound peptide from HST-2 gastric cancer cells in a reversed-phase HPLC column. In Fig. 1, a line indicates an acetonitrile concentration, and a curve indicates an absorbance at OD 210 in each concentration.

Fig. 2 is a graph showing a specific cytotoxicity of Tc-HST-2 when using an HST-2-derived HLA-bound peptide.

○ indicates a specific cytotoxicity when using HST-2.
♦ indicates a cytotoxicity when using an HLA-A31-positive KG-1 cell as a target T-cell.

All of the tests were conducted such that a ratio of target T-cells to CTL was 1:100.

Fig. 3 is an elution pattern of a 12th fraction showing a CTL inducibility shown in Fig.2 by a reversed-phase HPLC column. In Fig. 3, a line indicates an acetonitrile concentration, and a curve indicates an absorbance at OD 210 in each concentration.

Fig. 4 is a graph showing specific cytotoxicity of Tc-HST-2 when using an HST-2-derived HLA-bound peptide.

□ indicates specific cytotoxicity when using HST-2 as a target T-cell.
♦ indicates cytotoxicity when using an HLA-A31-positive CCRF-CEM cell as a targeT-cell.

All of the tests were conducted such that a ratio of target T-cells to CTL was 1:100.

Fig. 5 is a graph showing CTL inducibility of Tc-HST-2 measured by the TNF-releasing method when using Peptide 1 (SEQ ID NO:1), Peptide 1-9 (SEQ ID NO:2), Peptide 1-8 (SEQ ID NO:3) and Peptide 1-7 (SEQ ID NO:4).

The TNF-sensitive Wehi cells were impaired by the release of TNF, and incorporation of MTT by the residual cells is decreased. The peptide concentrations are 10 mM, 1 mM and 0.1 mM. In all of the tests, HST-2 was used as a target T-cell, and a ratio of target T-cells to CTL was 1:100.

■ indicates cytotoxicity when adding Peptide 1.
□ indicates cytotoxicity when adding Peptide 1-9.
∆ indicates cytotoxicity when adding Peptide 1-8.
○ indicates cytotoxicity when adding Peptide 1-7.
A broken line indicates cytotoxicity when not adding a peptide.

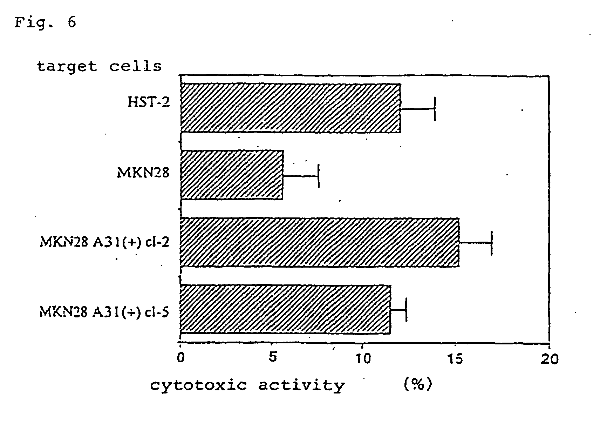

Fig. 6 is a graph showing cytotoxicity of TC-HST-2 when using MKN28 A31(+)cl-2 cells and MKN28 A31(+) cl-5 cells, which are sub-lines obtained by artificially expressing HLA-A31 and parental MKN28 gastric cancer cells. All of the tests were conducted such that the ratio of target T-cells to CTL was 1:100.

Detailed description of the invention

In order to identify a peptide which is a fragment of a gastric cancer antigen protein present in a human gastric cancer cell, the fragment being bound to an HLA molecule and capable of inducing a cytotoxic T-cell that targets the gastric cancer cell, it is necessary to establish (i) a HLA-type human cancer cell strain that can be grown in vitro in a large amount and (ii) a CTL cell strain which reacts with the above-mentioned cancer cell strain to restrict the HLA molecule and the T-cell receptor, exhibiting cytotoxicity, and which can be grown in a large amount in vitro.

For the HLA-type human gastric cancer cell strain (i) above, HST-2 cell established by the present inventors can be used (4). This cell strain is a gastric signet ring cell carcinoma strain formed from carcinomatous ascites of a patient suffering from a gastric cancer.

For the CTL cell strain (ii) above, Tc-HST-2 cell established by the present inventors can be used (4).

It has been described that this CTL cell strain specifically reacts with HST-2 cell and recognizes HST-2 antigen and HLA-A31 antigen at the same time (5).

The HST-2 cell can be used to obtain the HLA-bound peptide containing the gastric cancer antigen peptide capable of inducing the CTL activity of the Tc-HST-2 cell strain. That is, the HST-2 cell is incubated under ordinary incubation conditions, for example, in an RPMI1640 medium containing fetal bovine serum. Then, the culture is washed with phosphate-buffer saline (PBS). Thereafter, an HLA molecule-bound peptide present on the cell surface is extracted efficiently with a 0.1% trifluoroacetic acid (TFA) solution (6).

The gastric cancer antigen peptide capable of inducing the CTL activity of the Tc-HST-2 cell can be purified from the above-mentioned HLA-bound peptide through reversed-phase chromatography. The purification of this gastric cancer antigen peptide will be described later in the Examples.

The Tc-HST-2-reactive gastric cancer antigen peptide in the HLA-bound peptide fraction which is separated through reversed-phase chromatography can be identified by measuring the CTL inducibility of each fraction (6). That is, HLA-A31-positive HST-2 or KG-1 cell is radio-labeled with sodium chromate containing $^{51}$Cr, and is then mixed with the HLA-bound peptide separated through reversed-phase chromatography. The mixture is incubated at 37°C for 3 hours, and Tc-HST-2 is then added thereto. The resulting mixture is further incubated for 12 hours. The CTL inducibility can be detected by measuring the radio-activity of $^{51}$Cr released in the supernatant.

The amino acid sequence of the gastric cancer antigen peptide capable of inducing the CTL activity of the Tc-HST-2 cell separated through the reversed-phase chromatography can be determined in the usual manner using a solid-phase technique protein sequencer. For example, a peptide represented by SEQ ID NO:1 of the Sequence Listing can be mentioned as a peptide exhibiting the CTL inducibility, and can be used in the present invention. Further, one can also use a peptide represented by SEQ ID NO:2, obtained by deleting one amino acid from the C-terminal end of the peptide of SEQ ID NO:1. Also, one can use a peptide having the amino-acid sequence represented by SEQ ID NO:1, or SEQ ID NO:2 as a part of the overall amino-acid sequence.

The peptide of the present invention ranges from a peptide formed from a few amino acids to a peptide formed from many amino acids.

A peptide having an amino-acid sequence represented by SEQ ID NO:3 or SEQ ID NO:4 of the Sequence Listing cannot be used because it has no CTL inducibility (see the Examples below).

The method of producing the peptides hereof is not particularly limited. The peptide may be produced using a solid-phase peptide synthesis technique or a recombinant DNA technique.

For example, a CTL-inducible peptide that targets a gastric cancer cell as represented by SEQ ID NO:1 or SEQ ID NO:2 may be synthesized using a conventional solid-phase peptide synthesis technique. It may also be produced by incorporating DNA encoding the amino acids constituting this peptide into an appropriate expression vector, and incubating BCG bacteria or E coli transformed therewith.

DNA encoding such peptides can be deduced from the amino-acid sequence. Codons corresponding to each amino acid sequence are naturally well known to those skilled in the art.

All of the peptides in the present invention were purified by reversed-phase HPLC, and it was shown through reversed-phase HPLC that these are single ingredients. Then, the peptides were identified through mass spectrometer analysis, and were used in a CTL assay.

The peptide of the present invention can induce the CTL activity to the gastric cell-specific CTL strain Tc-HST-2. The CTL-inducibility can be measured by the above-mentioned chrome-releasing method or by a TNF-releasing method (7).

In the TNF-releasing method, the HLA-A31-positive HST-2 or KG-1 cell is mixed with the peptide of the present invention, and the mixture is incubated at 37°C for 3 hours. Then, Tc-HST-2 is added thereto, and the resulting mixture is further incubated for 12 hours. The activity of TNF released in the supernatant is measured (7) to detect the CTL inducibility.

For example, TNF-sensitive Wehi164 cells are mixed with a test sample, and the mixture is incubated for 24 hours. Then, an MTT [3-(4,5-dimethylthiazol-z-yl)-2,5-diphenyl tetrazolium bromide] solution is added to the culture, and the resulting mixture is further incubated for 3 hours. After formazan converted within mitochondria is dissolved in acid propanol, the amount thereof is measured in terms of an absorbance at OD570 to detect the TNF activity.

The peptide of the present invention, for example, the peptide having the amino-acid sequence represented by SEQ ID NO:1, can induce gastric cancer cell-specific CTL as a T-cell epitope. Accordingly, it can be expected to act as an agent for preventing or treating a human gastric cancer.

In use, the peptide of the present invention can be administered (i) as such, (ii) along with a pharmaceutically acceptable carrier and/or diluent, or (iii) along with adjuvants as required, using a syringe or through subcutaneous absorption from a mucous membrane, by spraying or the like. The carrier may for example be human serum albumin; the diluent may for example be PBS or distilled water.

The agent for preventing or treating human gastric cancer suitably contains from 0.01 to 100% by weight, preferably from 0.1 to 95% by weight, of the peptide which is a fragment of a gastric cancer antigen protein present in a human gastric cancer cell, the fragment being bound to an HLA molecule, suitably an HLA-A31 molecule, and capable of inducing a cytotoxic T-cell that targets the gastric cancer cell.

This agent is administered to an adult person at such a dose that the amount of the peptide of the present invention is between 0.01 mg and 100 mg in one administration. However, this range is only an indication, and may not be critical.

The formulation of the preparation is not particularly limited. A freeze-dried agent, or a granular agent containing a vehicle such as a saccharide, may be used. Such a preparation has no problematic acute toxicity in the above-mentioned administration.

Adjuvants which may be added to the agent of the present invention to increase the CTL inducibility include microorganisms such as BCG, ISCOM (8), saponin-type QS-21 (9), liposomes and aluminum hydroxide.

An immunological activator can be used as an adjuvant; for example lentinan, schizophyllan and picibanil. Cytokines that enhance growth or differentiation of T-cells, such as IL-2, IL-4, IL-12, IL-1, IL-6 and TNF can also be used as adjuvants.

It has been described (op cit supra, and 10) that the immune response of CTL or the like can be induced in vivo by the above-mentioned method.

It is possible that the antigen peptide hereof is added in vitro to cells collected from the patient or cells having the same HLA haplotype in order to present the antigen, after which these cells are administered to the blood vessel of the patient to effectively induce CTL within the body of the patient. It is also possible that the peptide hereof is added to peripheral blood lymphocytes of the patient and the peptide-containing lymphocytes are incubated in vitro to induce CTL in vitro, after which the resulting lymphocytes are returned to the blood vessel of the patient. Treatment through such cell transfer has been already practised as a cancer therapy, and it has become well known to those skilled in the art.

DNA encoding the gastric cancer antigen peptide of the present invention may be inserted into an appropriate vector. Viruses such as vaccinia virus or bacteria such as BCG, which contain this recombinant DNA, can effectively be used as a live vaccine for preventing or treating a human gastric cancer.

Thus, when (i) DNA encoding the peptide having the amino-acid sequence represented by SEQ ID NO:1, or (ii) DNA encoding a peptide having an amino-acid sequence obtained by modifying a part of the sequence of SEQ ID NO:1 (for example, a peptide having an amino-acid sequence represented by SEQ ID NO:2), is inserted into an antigen protein gene which is expressed in a recombinant virus or recombinant bacterium, this peptide sequence is expressed as a part of the antigen protein, then processed in the cells, and presented to the HLA antigen, making it possible to induce CTL that recognizes the the peptide.

The dose and the administration method of the agent are the same as those in usual vaccination or administration of BCG vaccine.

Examples

The present invention is illustrated more specifically by the following Examples.

Example 1

Purification of Tc-HST-2 response peptide from HST-2 and identification thereof:

HST-2 cells ($2 \times 10^5$ cells/ml) were inoculated in 50 ml of a 10% FCS-containing RPMI1640 medium (containing 2-mM glutamine, $5 \times 10^{-5}$M 2ME, 100 units/ml of penicillin, 100 μg/ml of streptomycin and 16mM $NaHCO_3$) in a 175-$cm^2$ plastic incubator (cat. No. 3028, manufactured by Falcon), and were incubated in the presence of a 5% $CO_2$ gas in air at 37°C for 4 days.

After completion of the incubation, the supernatant formed was removed, and the residue was washed with PBS (-). Then, 20 ml of 0.1% TFA solution were added, and the mixture was incubated at 4°C for 30 minutes. An HLA-bound peptide was released from the HLA molecule. The HLA-bound peptide released was centrifuged at 10,000xg for 15 minutes to separate the cell residue therefrom.

20 ml of the HLA-bound peptide solution obtained by incubating HST-2 as mentioned above were freeze-dried, and dissolved in 5 ml of a 0.1% TFA solution. 1 ml of this HLA-bound peptide solution was treated with a reversed-phase HPLC column (μ BONDSHERE, 5 μ C18-300 A, 19 mm x 150 mm). The reversed-phase HPLC column had been equilibrated with 0.1% TFA-containing distilled water in advance, and the desired substance was eluted by increasing the concentration of acetonitrile in 0.1% TFA linearly from 0 to 80%. This procedure was conducted at a flow rate of 1 ml/min. The eluent was collected in amounts of 1 ml each.

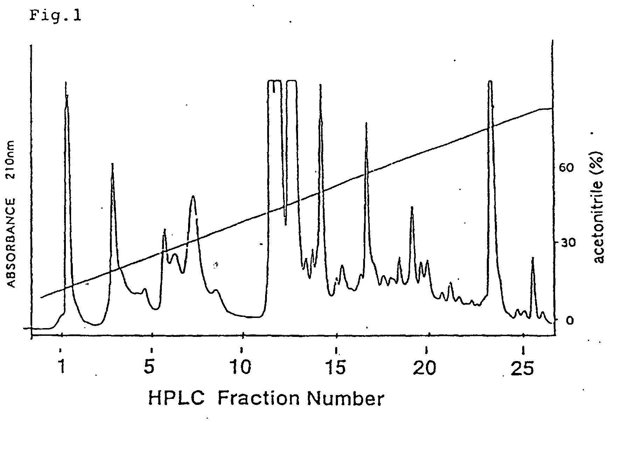

The above procedure was repeated for a total of 10 times. Fractions that showed the same retention time were collected, and freeze-dried. No significant difference was found in elution patters of HPLC in the above-mentioned procedures. The elution patterns of HPLC are shown in Fig. 1.

The CTL inducibility of the HPLC elution fractions against Tc-HST-2 cells was measured as follows. $1 \times 10^6$ HST-2 or KG-1 cells expressing HLA-A31 antigen as target T-cells were suspended in a 10% FCS-containing RPIM medium such that the number of cells reached $1 \times 10^7$ cells/ml. To the suspension were added 100 μl of a mixture of sodium chromate containing $^{51}$Cr and PBS, and the resulting mixture was incubated in the presence of 5% $CO_2$ at 37°C for 3 hours for $^{51}$Cr-labelling. The $^{51}$Cr-labelled cells ($1 \times 10^4$ cells) were suspended in 100 μl of the solution. 3 ml of the solution containing the HLA-bound peptide separated through HPLC were added, and the mixture was further incubated under the same conditions for 3 hours, whereby the HLA-bound peptide was bound to the HLA molecule.

Subsequently, Tc-HST-2 cells ($2.5 \times 10^5$ cells/ml) were added thereto in an amount of 100 μl, and the mixture was incubated under the same conditions for 12 hours. After 12 hours of incubation, 100 μl of the supernatant formed were collected, and the radioactivity of $^{51}$Cr released in this supernatant was measured using a γ-counter to measure the CTL activity.

The specific cytotoxicity was calculated using the following expression.

$$\text{Specific cytotoxicity} = \frac{\text{measured value of each well - minimum release value}}{\text{maximum release value - minimum release value}} \times 100$$

wherein:

the minimum release value indicates the measured value of $^{51}$Cr naturally released from target T-cells in a well filled with target T-cells alone,

the maximum release value indicates the measured value of $^{51}$Cr when the cells are destroyed by adding surfactant 1% Triton X-100 to target T-cells.

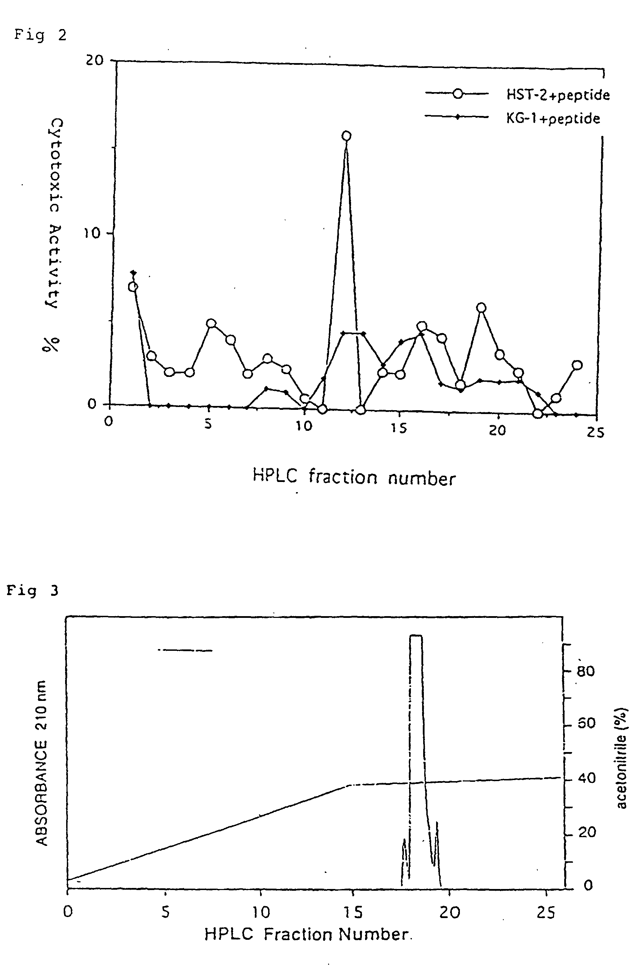

The results are shown in Fig. 2. As is clear from Fig. 2, the CTL inducibility was observed in the 12th fraction eluted through HPLC.

The 12th fraction of which the CTL inducibility of Tc-HST-2 had been identified was further treated with a reversed-phase HPLC column (μ BONDSHERE, 5 μ C18-300A, 19 mm x 150 mm). The reversed-phase HPLC column had been equilibrated with 0.1% TFA-containing distilled water in advance, and then the desired substance was eluted by increasing the concentration of acetonitrile in 0.1% TFA-containing eluent linearly from 0 to 40%. This procedure was conducted at a flow rate of 1 ml/min. The eluent was collected in amounts of 1 ml each. The above procedure was repeated for a total of 10 times. Fractions that showed the same retention time were collected, and freeze-dried. No significant difference was found in elution patterns of HPLC in the above-mentioned procedures. The elution patterns of HPLC are shown in Fig. 3.

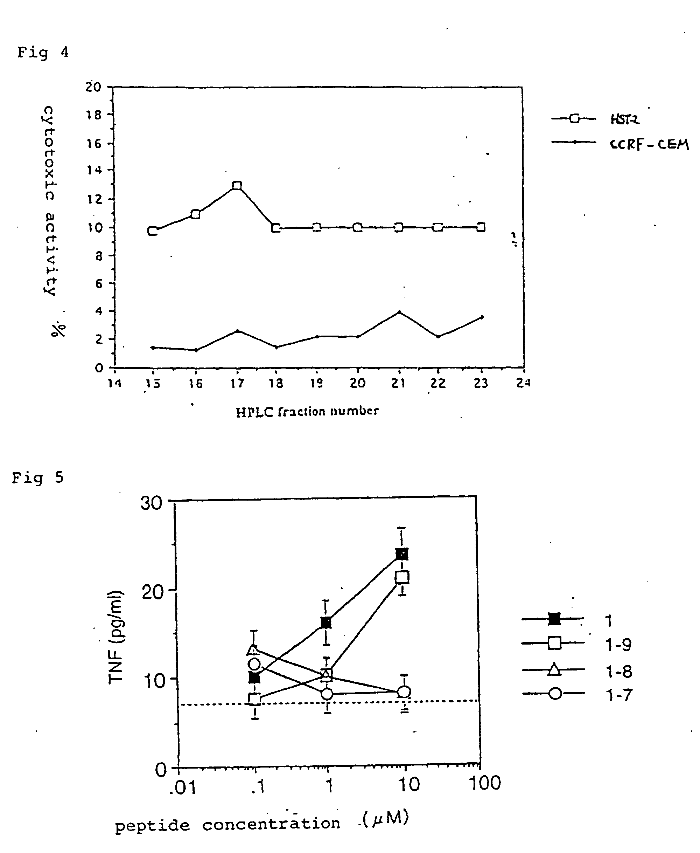

The CTL inducibility of Tc-HST-2 cells in the HPLC elution fractions was measured by the above-mentioned method. The results are shown in Fig. 4. As is clear from Fig. 4, the CTL inducibility was identified in the 17th fraction eluted through HPLC.

The 17th fraction of which the CTL inducibility had been identified was separated with a reversed-phase HPLC column for analysis (ZORBAX, ODS column, 4 mm x 250 mm). The reversed-phase HPLC column had been equilibrated with 0.1% TFA-containing distilled water in advance, and then the desired substance was eluted by increasing the concentration of acetonitrile in 0.1% TFA containing eluent linearly from 0 to 60%. This procedure was conducted at a flow rate of 1 ml/min to obtain a main peak.

This peak was taken out, and introduced into a protein sequencer (477A, manufactured by ABI). The amino-acid sequence was determined by the Edman degradation method. The amino-acid sequence from the N-terminal is represented by SEQ ID NO:1 of the Sequence Listing.

### Example 2

Synthesis of a gastric cancer antigen peptide and measurement of CTL inducibility:

Synthetic Peptide 1 having the same amino-acid sequence as that represented by SEQ ID NO:1, and three types of peptides containing from 9th to 7th amino acids from the N-terminal of this Synthetic Peptide 1, namely, Peptide 1-9 (SEQ ID NO:2), Peptide 1-8 (SEQ ID NO:3), and Peptide 1-7 (SEQ ID NO:4), were synthesized using an automated peptide synthesizer (477A, manufactured by ABI) according to the protocol in the manual of the same machine.

These synthetic peptides were cut from the resin, and reprecipitated with a mixture of TFA and dry ether. They were identified to be single components through reversed-phase HPLC, and were further identified using a mass spectrometer. Subsequently, they were subjected to CTL assay using a TNF-releasing method.

1 x $10^4$ HST-2 cells expressing HLA-A31 antigen were suspended as target T-cells in 100 μl of the solution, and 5 μl of each of the above-mentioned four synthetic peptides were added thereto such that the final concentration reached 10 μM, 1 μM or 0.1 μM. The mixture was further incubated under the same conditions for 3 hours to bind the HLA-bound peptide to the HLA molecule. Subsequently, Tc-HST-2 cells (1 x $10^6$ cells/ml) were added thereto in an amount of 100 μl, and the resulting mixture was further incubated for 12 hours under the same conditions.

After 12 hours of incubation, 30 μl of the supernatant formed was collected, and the TNF activity released in the supernatant was measured to detect the CTL inducibility. The TNF activity was. measured by the following method.

7 x $10^4$ Wehi 164 cells, which are TNF-sensitive cells, were suspended in 120 μl of the solution, and 30 μl of the above-obtained supernatant were added. The mixture was incubated for 24 hours. After the completion of the incubation, 5 mg/ml of MTT were added to each well in amounts of 10 μl each, and the mixture was further incubated for 3 hours. Subsequently, 150 μ of propanol containing 0.01% hydrochloric acid were added to solubilize formazan formed during the incubation. Then, the absorbance was measured at a wavelength of 570 nm to measure the TNF activity.

The results are shown in Fig. 5. As is clear from Fig. 5, the CTL inducibility of TC-HST-2 was identified in Synthetic Peptides 1 and 1-9.

### Example 3

Expression of a gastric cancer antigen peptide in human MKN28 gastric cancer cell strain:

In order to identify whether or not a gastric cancer antigen peptide recognized by TC-HST-2 was expressed in gastric cancer cell strains other than HST-2, the cytotoxicity given by TC-HST-2 to MKN28, which is an HLA-A31-negative human gastric cancer cell strain, was detected by the $^{51}$Cr-releasing method. The detection of the CTL activity by the $^{51}$Cr-releasing method was conducted in the same manner as in Example 1.

The results are shown in Fig. 6. TC-HST-2 did not exhibit cytotoxicity to the MKN28 cells which did not express HLA-A31. On the other hand, TC-HST-2 exhibited significant cytotoxicity to MKN28 A31(+)cl-2 and MKN28 A31(+)cl-5, sub-lines of MNK28 formed by transfecting plasmid pBJ-A31 containing HLA-A31 gene into MKN28 through electroporation to express HLA-A31, as shown in Fig. 6.

From the above-mentioned results, it was identified that the gastric cancer antigen peptide recognized by TC-HST-2 was also expressed in MKN28, and that this peptide was bound to the HLA-A31 molecule and recognized by TC-HST-2.

### Effects of the Invention

The peptide of the present invention can induce CTL to human gastric cancer cells _in vivo_ or _in vitro_, and this peptide itself and the agent comprising this peptide can be expected to perform as an agent for preventing or treating a human gastric cancer. Further, a virus or a bacterium containing recombinant DNA encoding the peptide capable of inducing CTL to the human gastric cancer cells in vivo or in vitro should exhibit a pharmaceutical effect as a vaccine for preventing or treating human gastric cancer.

References

1. Medical Immunology, revised 3rd edition, compiled by Kikuchi Kokichi
2. Human Leucocyte Antigen; refer to Modern Immunology, 2nd edition, compiled by Yamamura Yuichi and Tada Tomio
3. J. Immunol. Meth. 154, 235-243, (1992), and Cancer 75, 1484-1489, (1995).
4. J. Immunol. Meth., vol. 154, pp. 235 - 243 (1992); and Jpn. J. Cancer Res., vol. 84, pp. 906 - 913 (1993)
5. Cancer, vol. 75, pp. 1484 - 1489 (1995)
6. Science, vol. 249, pp. 283 - 287 (1990).
7. Immunogenetics, vol. 35, p. 145 (1992).
8. Nature, vol. 344, p. 873 (1990)
9. J. Immunol., vol. 148, p. 1438 (1992)
10. Science, vol. 255, p. 333 (1992)

Sequence Listing:

SEQ ID NO:1
Length of sequence: 10
Type of sequence: amino acid
Topology: linear
Type of sequence: peptide
Origin: human
Sequence:

```
Tyr Ser Trp Met Asp Ile Ser Cys Trp Ile
 1               5                   10
```

SEQ ID NO:2
Length of sequence: 9
Type of sequence: amino acid
Topology: linear
Type of sequence: peptide
Origin: human
Sequence:

```
Tyr Ser Trp Met Asp Ile Ser Cys Trp
 1               5
```

SEQ ID NO:3
Length of sequence: 8
Type of sequence: amino acid
Topology: linear
Type of sequence: peptide
Origin: human
Sequence:

```
Tyr Ser Trp Met Asp Ile Ser Cys
 1               5
```

SEQ ID NO:4
Length of sequence: 7
Type of sequence: amino acid
Topology: linear
Type of sequence: peptide
Origin: human
Sequence:

```
Tyr Ser Trp Met Asp Ile Ser
 1               5
```

## Claims

1. A composition comprising a peptide which is a fragment of a gastric cancer antigen protein present in a human gastric cancer cell, said peptide being bound to HLA-A31 and capable of inducing a cytotoxic T cell response that targets the gastric cancer cell, wherein said peptide has an amino acid sequence represented by SEQ ID NO:1 or SEQ ID NO:2.

2. An agent for preventing or treating a human gastric cancer, said agent comprising a peptide which is a fragment of a gastric cancer antigen protein present in a human gastric cancer cell, said peptide being bound to HLA-A31 and capable of inducing a cytotoxic T cell response that targets the gastric cancer cell, wherein said peptide has an amino acid sequence represented by SEQ ID NO:1 or SEQ ID NO:2.

3. A peptide which is a fragment of a gastric cancer antigen protein present in a human gastric cancer cell, which peptide, when bound to HLA-A31 is capable of inducing a cytotoxic T cell response that targets the gastric cancer cell, wherein said peptide has an amino acid sequence represented by SEQ ID NO:1 or SEQ ID NO:2.

4. DNA encoding the peptide of claim 3.

5. A vaccine for preventing or treating a human gastric cancer, said vaccine containing a recombinant virus or a recombinant bacterium having the DNA of claim 4.

## Patentansprüche

1. Zusammensetzung, die ein Peptid enthält, welches ein Fragment eines in einer menschlichen Magenkrebszelle vorhandenen Magenkrebsantigenproteins ist, wobei das Peptid an HLA-A31 gebunden ist und die Antwort einer cytotoxischen T-Zelle, deren Zielzelle eine Magenkrebszelle ist, induzieren kann, wobei das Peptid eine Aminosäuresequenz hat, die durch SEQ ID NO:1 oder SEQ ID NO:2 dargestellt ist.

2. Mittel zum Verhindern oder Behandeln eines menschlichen Magenkrebses, wobei das Mittel ein Peptid enthält, welches ein Fragment eines in einer menschlichen Magenkrebszelle vorhandenen Magenkrebsantigenproteins ist, wobei das Peptid an HLA-A31 gebunden ist und die Antwort einer cytotoxischen T-Zelle, deren Zielzelle eine Magenkrebszelle ist, induzieren kann, wobei das Peptid eine Aminosäuresequenz hat, die durch SEQ ID NO:1 oder SEQ ID NO:2 dargestellt ist.

3. Peptid, welches ein Fragment eines in einer menschlichen Magenkrebszelle vorhandenen Magenkrebsantigenproteins ist, wobei das Peptid, wenn es an HLA-A31 gebunden ist, die Antwort einer cytotoxischen T-Zelle, deren Zielzelle die Magenkrebszelle ist, induzieren kann, wobei das Peptid eine Aminosäuresequenz hat, die durch die SEQ ID NO:1 oder SEQ ID NO:2 dargestellt ist.

4. DNA, die das Peptid nach Anspruch 3 kodiert.

5. Impfstoff zum Verhindern oder Behandeln eines menschlichen Magenkrebses, wobei der Impfstoff einen rekombinanten Virus oder ein rekombinantes Bakterium mit der DNA nach Anspruch 4 enthält.

**Revendications**

1. Composition comprenant un peptide, qui est un fragment d'une protéine antigénique d'un cancer gastrique, présente dans une cellule d'un cancer gastrique humain, ledit peptide étant lié à HLA-A31 et étant capable d'induire une réponse cytotoxique des lymphocytes T dirigée contre la cellule cancéreuse gastrique, dans laquelle ledit peplide a la séquence d'acides aminés représentée par SEQ ID N°1 ou SEQ ID N°2.

2. Agent pour la prévention ou le traitement d'un cancer gastrique humain, ledit agent comprenant un peptide, qui est un fragment d'une protéine antigénique d'un cancer gastrique, présente dans une cellule d'un cancer gastrique humain, ledit peptide étant lié à HLA-A31 et étant capable d'induire une réponse cytotoxique des lymphocytes T dirigée contre la cellule cancéreuse gastrique, dans laquelle ledit peptide a la séquence d'acides aminés représentée par SEQ ID N°1 ou SEQ ID N°2.

3. Peptide, qui est un fragment d'une protéine antigénique d'un cancer gastrique, présente dans une cellule d'un cancer gastrique humain, ledit peptide, quand il est lié à HLA-A31, étant capable d'induire une réponse cytotoxique des lymphocytes T dirigée contre la cellule cancéreuse gastrique, ledit peptide ayant la séquence d'acides aminés représentée par SEQ ID N°1 ou SEQ ID N°2.

4. ADN codant pour le peptide de la revendication 3.

5. Vaccin pour la prévention ou le traitement d'un cancer gasrrique humain, ledit vaccin contenant un virus recombinant ou une bacténie recombinante possédant l'ADN de la revendication 4.

Fig.1

Fig 2

Fig 3

Fig 4

Fig 5

Fig. 6

target cells

HST-2

MKN28

MKN28 A31(+) cl-2

MKN28 A31(+) cl-5

0　　　　　5　　　　10　　　　15　　　　20

cytotoxic activity　　　　(%)

14